# FASCICULE DE BREVET EUROPEEN

(11) **EP 1 218 058 B1**
(45) Date de publication et mention de la délivrance du brevet: **30.11.2005**
(21) Numéro de dépôt: 00964342.0
(22) Date de dépôt: 21.09.2000
(51) Int. Cl.: A61N 1/24, A61N 1/20, A61N 1/16

(54) **DISPOSITIF A CHAMPS ELECTROGALVANIQUES POUR LE TRAITEMENT OU LA PROTECTION D'ORGANISMES VIVANTS**
VORRICHTUNG ZUM BEHANDELN ODER SCHÜTZEN VON LEBEWESEN
ELECTROGALVANIC FIELD DEVICE FOR TREATING OR PROTECTING LIVING ORGANISMS

(30) Priorité: 24.09.1999 FR 9912186
(43) Date de publication de la demande: 03.07.2002
(73) Titulaire: Bricot, Bernard, 13008 Marseille (FR)
(72) Inventeur: Bricot, Bernard, 13008 Marseille (FR)
(74) Mandataire: Marek, Pierre
(86) Numéro de dépôt international: PCT/FR2000/002621
(87) Numéro de publication internationale: WO 2001/021252

(56) Documents cités:
- EP-A- 0 144 610
- WO-A-93/24175
- CH-A- 183 323
- FR-A- 2 505 660
- FR-A- 2 642 654
- US-A- 4 616 654

## Description

La présente invention concerne un dispositif produisant un champ électrogalvanique ou un champ électromagnétique et destiné, selon une première application, à être placé à proximité ou au contact de certaines parties ou zones réflexes d'un organisme vivant (personne ou animal), en vue de leur traitement ou rééquilibration par stimulation ou information ; on parle alors de biostimulation. Selon une deuxième application, ce dispositif est destiné à être placé sur des appareils électriques ou à proximité de ceux-ci, afin de neutraliser, les effets délétères de ces derniers sur l'organisme ; on parle alors de neutralisation ou de "bio-neutralisation".

On sait qu'il existe un "invariant postural" qui représente la position idéale du corps dans l'espace, à un moment donné de l'évolution phylogénétique de l'individu. On a pu ainsi définir des critères de normalité en posturologie clinique qui ont conduit à constater que plus de 90 % des individus présentent un déséquilibre postural.

Les troubles et affections engendrés par les désordres posturaux sont extrêmement nombreux. Par exemple, les désordres ou troubles du rachis peuvent engendrer des rachialgies (cervicalgies, lombalgies, dorsalgies, etc.), des troubles de la statique (scolioses, cyphoses, bascules du bassin, etc.), des céphalées (céphalées occipitales, névralgies d'Arnold, céphalées globales, etc.), des névralgies cervico-brachiales, des scapulalgies et des myalgies, des douleurs lombaires, des sciatiques et autres perturbations influencées par les désordres rachidiens en raison du rapport intime existant entre la colonne vertébrale et le système nerveux autonome qui est en rapport avec tous les organes.

Un grand nombre de pathologies "fonctionnelles" sont ainsi provoquées ou, tout au moins, nettement influencées par les désordres posturaux.

Il est bien établi de nos jours, qu'il est préférable de traiter les déséquilibres posturaux à leur source et non pas uniquement par leurs symptômes. On sait que la peau est capable de transmettre au cerveau certaines informations qu'elle reçoit (voies lemniscales et extralemniscales) et que le cerveau est capable d'analyser ces informations. On sait également qu'il est possible de traiter des troubles et des affections dûs au déséquilibre rachidien, en corrigeant certaines zones réflexes cutanées ou certains points d'acupuncture à l'aide de micro-courants (diascope, punctoscope...), de rayonnements laser ou de champs magnétiques ; toutefois, il existe peu d'appareillages destinés à corriger ces zones de façon permanente. Par exemple, il est notamment décrit dans le document FR-2 255 922A, des dispositifs de traitement par galvano-thérapie et acupuncture, comprenant deux électrodes et un électrolyte dans lequel est plongée une partie du corps du patient. Ces dispositifs sont très encombrants, du fait qu'ils nécessitent une cuve pour immerger la partie du corps du patient à traiter, et ils sont d'un maniement complexe pour lequel les connaissances d'un technicien qualifié sont requises. L'effet de ces courants reste donc sporadique, la durée des séances d'électro-stimulation et le résultat recherché incertains, dû notamment aux variations de distances pouvant exister entre la partie du corps à traiter et les électrodes de stimulation pendant la séance de traitement.

Pour surmonter ces inconvénients, il a été proposé, dans le document EP-0.065.473, des semelles de reprogrammation posturale, lesquelles en polarisant les champs de radiations terrestres, appliquent sur les zones réflexes des pieds, un flux d'énergie ondulatoire polarisé lorsqu'elles sont en contact direct avec la peau de la voûte plantaire.

Ces semelles de reprogrammation posturale à champs polarisants donnent de bons résultats, mais on a souhaité pouvoir disposer d'un dispositif ayant une action plus complète et plus rapide et que l'on ne soit pas obligé de placer au contact de la peau.

Un dispositif tel que défini dans le préambule de la revendication 1 est connu du document FR-A-2 642 654.

La présente invention a pour but de remédier aux inconvénients des différents appareils et dispositifs connus pour le traitement des zones réflexes ou autres zones cutanées, au moyen de champs électriques ou de champs magnétiques, en proposant un dispositif facile à mettre en oeuvre, efficace même à distance et peu encombrant.

Le dispositif de biostimulation et de bio-neutralisation selon l'invention est défini dans la revendication 1.

Ce dispositif est encore remarquable par son utilisation comme moyen de biostimulation de différentes parties d'un organisme vivant, ou par son utilisation comme moyen de neutralisation des effets délétères des appareils utilisant une énergie électrique pour leur fonctionnement.

Dans le premier cas, le dispositif de biostimulation est placé à proximité de la zone réflexe ou de la partie de l'organisme vivant à traiter, en utilisant, de préférence, différents moyens de contention, ou appliqué sur ladite zone-réflexe. Dans le deuxième cas, le dispositif de neutralisation est fixé sur l'appareil utilisant l'énergie électrique.

Grâce à la nature électrochimique différente des deux métaux constituant le dispositif selon l'invention, un champ électrogalvanique ou un champ: électromagnétique est généré, qui rééquilibre, stimule, ou informe les zones réflexes ou qui interagit avec celles-ci, à proximité desquelles il est placé, et/ou neutralise les micro-courants perturbateurs comme, par exemple, l'électrogalvanisme buccal, les champs perturbateurs des téléphones portables sur lesquels il est placé.

Grâce à sa petite taille, le dispositif selon l'invention peut être inclus dans des moyens de contention variés communément utilisés pour l'habillement ou les soins des personnes tels que chaussures, ceintures, corsets, bandages, pansements, etc., ou dans différentes substances, telles que résines élastomères, substances plastiques ou apparentées, verre ionomère, composite, etc... Il peut aussi être placé au dos d'une montre, ou directement sur un téléphone portable ou sur sa batterie.

Selon une mise en oeuvre particulièrement avantageuse, l'invention peut être constituée par une semelle apte à se loger de manière amovible dans une chaussure et entre les couches constitutives superposées de laquelle est incorporé un petit générateur de champs électrogalvaniques ou électromagnétique réalisé sous forme d'une plaquette plate de contour circulaire, carré, rectangulaire ou autre.

Un avantage majeur de ce dispositif découle du fait qu'il n'est pas nécessaire de le porter au contact direct de la peau, de sorte qu'il peut être inclus et protégé dans un moyen de contention couramment utilisé, sans transformation notable de ce dernier, tout en restant pleinement efficace. Par exemple, lorsqu'il est placé au niveau de chaque pied (ou d'un seul selon le cas), le champ symétrique (ou asymétrique, en fonction des cas), ainsi généré, permet d'équilibrer les chaînes musculaires posturales, ce qui engendre :
- un meilleur équilibre du corps ;
- moins de tensions musculaires ;
- moins de blocages vertébraux ;
- une meilleure posture et une meilleure stabilité ;
- moins de douleurs vertébrales et rhumatismales ;
- etc.

Lorsqu'il est exécuté sous forme de semelles de biostimulation, permettant un rééquilibrage des chaînes musculaires posturales, l'action de ces semelles est plus complète et plus rapide, par rapport à celle des semelles à champs polarisants décrites dans le document EP-0.065.473. Notamment, elles agissent encore mieux et plus rapidement que ces dernières dans toutes les pathologies douloureuses vertébrales et rhumatismales ou dans les pathologies à composante statique ou posturale (hanches, genoux, pieds) et dans toutes les pathologies déformatives de la colonne vertébrale (scoliose) et des membres (défauts d'axes, pieds qui tournent).

Le dispositif de biostimulation selon l'invention peut être collé directement dans des chaussures, notamment l'été, ou dans les chaussures utilisées par les sportifs de haut niveau ou par les danseurs et danseuses.

Selon le dessin annexé :
La figure 1 est une vue de face d'un exemple d'exécution intéressant, bien que nullement limitatif, du dispositif à champs électrogalvaniques ou à champs électromagnétiques selon l'invention.
La figure 2 est une vue en coupe diamétrale de ce dispositif.
La figure 3 est une vue de face d'un exemple de moyen de contention incluant le dispositif selon l'invention, ce moyen étant, suivant cet exemple, constitué par une semelle destinée à être logée dans une chaussure.
La figure 4 est une vue en coupe longitudinale de cette semelle.
La figure 5 est une vue de face d'un autre exemple de réalisation du dispositif à champs électrogalvaniques ou à champs électromagnétiques de l'invention.
La figure 6 est une vue en coupe diamétrale de ce dispositif.
La figure 7 est une vue en coupe longitudinale d'une semelle comportant un générateur de champs électrogalvaniques représenté aux figures 5 et 6.
La figure 8 est une vue en coupe diamétrale d'un troisième exemple de réalisation du dispositif à champs électrogalvaniques ou à champs électromagnétiques de l'invention.

On se reporte auxdits dessins pour décrire des formes de réalisation avantageuses, quoique non limitative, du dispositif de biostimulation ou de neutralisation selon l'invention.

Ce dispositif est remarquable par le fait qu'il est remarquable par le fait qu'il est réalisé sous forme d'une plaquette plate 1 constituée d'une partie centrale 2 encastrée dans une partie périphérique 3, lesdites parties centrale 2 et périphérique 3 étant réalisées dans des métaux différents ou dans des alliages de métaux différents, la plaquette biostimulante ainsi réalisée pouvant avoir une forme carrée, rectangulaire, circulaire ou autre.

Selon la forme d'exécution illustrée, le générateur de champs ou courants de l'invention se présente sous forme d'un petit disque plat 1 constitué d'une pastille circulaire 2 encastrée, concentriquement, dans une rondelle 3, cette pastille circulaire et cette rondelle étant réalisées dans des métaux ou dans des alliages de métaux de nature et de potentiels différents.

De façon très avantageuse, ces métaux peuvent être le zinc et le cuivre. Leur association permet de constituer une pile ou générateur de courant électrique produisant un champ électrogalvanique ou électromagnétique utilisable pour rééquilibrer les zones réflexes cutanées, ou pour contrer les effets délétères des micro-courants électriques perturbateurs.

La pastille centrale 2 peut être exécutée en cuivre et la rondelle périphérique 3 en zinc, ou vice-versa.

D'autre part, le dispositif selon l'invention peut être constitué de plus de deux éléments métalliques de nature différente (métaux différents ou alliages de métaux différents).

Selon l'invention, le petit générateur de courants électrogalvanique ou électromagnétique est placé à proximité des zones réflexes ou des parties du corps des organismes visants à traiter, ou appliqué sur ces zones réflexes ou parties. On précise que l'expression "à proximité" doit être comprise comme désignant toutes distances auxquelles les zones réflexes ou autres parties du corps peuvent réagir aux effets des courants ou champs électrogalvaniques ou électromagnétiques du générateur décrit dans le présent exposé.

Le dispositif à champs électrogalvaniques ou à champs électromagnétiques selon l'invention, peut être inclus dans des moyens de contention 4 qui peuvent être constitués par des articles divers d'utilisation courante, tels que semelles, chaussures, ceintures, corsets, bandages, pansements ou autres destinés à être appliqués contre certaines parties du corps des organismes vivants.

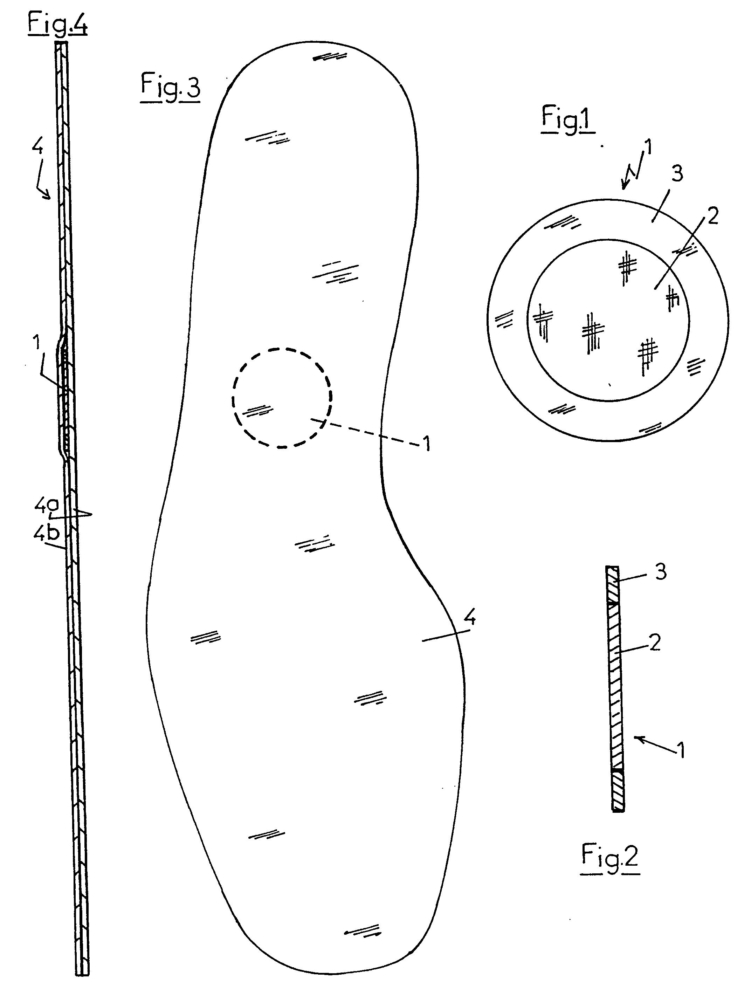

On a illustré, aux figures 3 et 4, un mode d'exécution avantageux de l'invention sous forme d'une semelle 4 de biostimulation constituée de deux couches ou épaisseurs superposées 4a, 4b, découpées dans un ou des matériaux tels que, par exemple : cuir, simili-cuir, peau, simili-peau, carton, tissus, feutre, liège, matière plastique, etc., entre lesquelles est incorporé, selon tout procédé convenable et à un emplacement correspondant à la région où se trouve(nt) la ou les zones-réflexes à traiter, un petit générateur de champs électrogalvaniques ou électromagnétiques 1 réalisé de la manière indiquée précédemment sous forme d'une petite plaque plane, de forme circulaire ou autre dont les composants 2 et 3 sont de préférence, disposés dans un même plan.

Compte tenu du fait que les points de départ podaux des principales chaînes proprioceptives ascendantes agissant sur les ensembles musculaires staturaux se trouvent dans la région de la voûte plantaire, et que la peau du pied est particulièrement sensible à l'action des champs électrogalvaniques et aux stimulations fréquentielles, la zone centrale de la plante du pied est une partie du corps particulièrement équilibrante, de sorte que le petit générateur de champs électrogalvaniques 1 peut être positionné dans la zone médiane de la semelle 4.

Le petit générateur de champs électrogalvanique ou champs électromagnétiques 1 peut être collé sur la couche inférieure 4a de la semelle 4, au moyen d'une pellicule de matière adhésive 5, par exemple constituée par une colle au néoprène.

De préférence, une pellicule de matière imperméable 6 recouvre la face du générateur 1 destinée à être orientée en direction de la zone cutanée à stimuler, afin d 'éviter la corrosion dudit générateur, cette pellicule étant fixée sur ladite face, par exemple au moyen d'une colle au néoprène.

On comprend que la semelle selon l'invention permet de soumettre les zones-réflexes de la voûte plantaire aux effets biostimulants des champs ou courants générés par le petit générateur 1.

Bien entendu, le petit générateur de champs électrogalvaniques ou électromagnétiques peut être logé à tout autre endroit convenable de la semelle, suivant l'emplacement de la ou des zones-réflexes qu'il est nécessaire de stimuler.

Les semelles selon l'invention peuvent se loger de façon amovible dans les chaussures des utilisateurs ou constituer directement les semelles permanentes de chaussures de traitement.

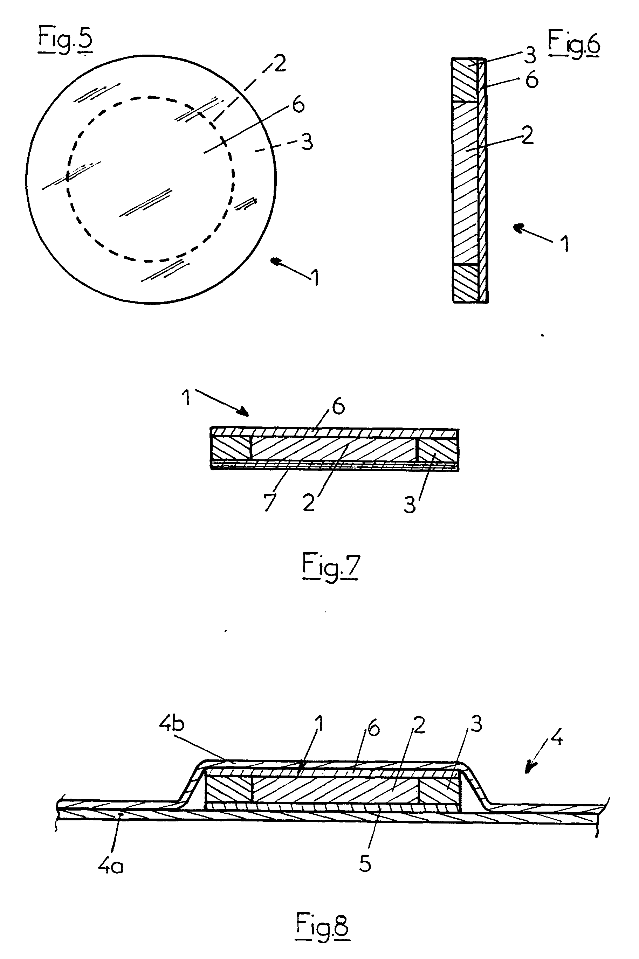

Dans le premier cas, la face du générateur 1 destinée à être fixée, de manière amovible, sur une surface réceptrice de la chaussure ou autre moyen de contention, peut être recouverte d'une pellicule auto-adhésive 7, fixée sur ladite face, par exemple au moyen d'une colle au néoprène (figure 7).

À titre indicatif seulement, ce petit disque plat peut avoir un diamètre de l'ordre de 10 à 50 mm et une épaisseur de l'ordre de 1 mm.

On observe que, de la taille et de l'épaisseur des métaux ou alliages de métaux, dépend la fréquence de charge et de décharge du générateur.

Il est facilement compréhensible que les champs électriques émis par le dispositif selon l'invention, peuvent être utilisés pour informer certaines zones cutanées dont on connaît l'aptitude à transmettre l'information au centre supérieur (voies lemniscale et extralemniscale), de sorte que ces champs électriques peuvent interagir avec les flux de potentiels présents dans le corps humains, lesquels peuvent être stimulés en cas de défaillance ou au contraire, informer pour neutraliser l'effet délétère de certains courants (électrogalvanisme buccal, champs perturbateurs des téléphones portables, etc.).

L'invention peut-être utilisée pour réaliser un moyen de traitement de différentes parties d'un organisme vivant, et /ou pour réaliser un moyen de neutralisation des effets délétères des appareils utilisant une énergie électrique pour leur fonctionnement.

## Revendications

1. Dispositif de biostimulation d'organismes vivants, et/ou de neutralisation des effets délétères d'appareils, composé par un générateur de champs électromagnétiques réalisé sous forme d'une plaquette plate (1) comprenant au moins deux composants métalliques (2, 3) constitués par des métaux différents, **caractérisé en ce que** la plaquette plate (1) est constituée d'une partie centrale (2) encastrée dans une partie périphérique (3), lesdites parties centrale (2) et périphérique (3) étant réalisées dans des métaux.

2. Dispositif selon la revendication 1, **caractérisé en ce que** la plaquette plate (1) est réalisé sous forme d'un petit disque (1) constitué d'une pastille circulaire (2) encastrée, concentriquement, dans une rondelle (3), lesdites pastille et rondelle étant réalisées dans des métaux différents ou dans des alliages de métaux différents.

3. Dispositif selon l'une des revendications 1 ou 2, **caractérisé en ce que** la partie centrale (2) est réalisée en cuivre, tandis que la partie périphérique (3) est exécutée en zinc, ou vice-versa.

4. Dispositif suivant l'une quelconque des revendications 1 à 3, **caractérisé en ce que** les composants (2, 3) de la plaquette plate (1) sont disposés dans un même plan.

5. Dispositif suivant l'une quelconque des revendications 1 à 4, **caractérisé en ce qu'**il est logé dans une semelle (4) apte à se loger de manière amovible dans une chaussure.

6. Dispositif selon l'une quelconque des revendications 1 à 4, **caractérisé en ce qu'**il est logé dans un moyen de contention (4) constitué par un article d'usage courant destiné à être appliqué contre une partie du corps des organismes vivants, tels que chaussures, ceintures, corsets, bandages, pansements.

7. Dispositif selon l'une quelconque des revendications 1 à 6, **caractérisé en ce que** sa face appliquée ou destinée à être appliquée sur un moyen de contention (4) est recouverte d'une pellicule de matière adhésive (5), ou d'une pellicule auto-adhésive (7).

8. Dispositif selon l'une quelconque des revendications 1 à 7, **caractérisé en ce que** sa face destinée à être orientée en direction de la zone cutanée à stimuler, est recouverte d'une pellicule de matière imperméable (6), fixée sur ladite face, par exemple au moyen d'une colle ou néoprène.

9. Dispositif constitué par une semelle (4) destinée à être appliquée sous la plante des pieds comprenant deux couches superposées (4a, 4b) entre lesquelles est incorporé le dispositif selon l'une quelconque des revendications 1 à 8.

10. Dispositif selon l'une des revendications 5 ou 9, **caractérisé en ce que** le générateur de champs est positionné dans la zone médiane de la semelle (4).

## Patentansprüche

1. Vorrichtung zur Biostimulation von lebenden Organismen und/oder zur Neutralisierung der schädlichen Auswirkungen von Einrichtungen, die aus einem Generator für elektromagnetische Felder aufgebaut ist, der in Form einer flachen Platte (1) ausgeführt ist, umfassend wenigstens zwei metallische Komponenten (2, 3), die aus verschiedenen Metallen bestehen, **dadurch gekennzeichnet, dass** die flache Platte (1) aus einem mittleren Teil (2) gebildet ist, der in einen Umfangsteil (3) eingebettet ist, wobei der mittlere Teil (2) und der Umfangsteil (3) aus Metallen ausgeführt sind.

2. Vorrichtung nach Anspruch 1, **dadurch gekennzeichnet, dass** die flache Platte (1) in Form einer kleinen Scheibe (1) ausgeführt ist, die aus einem kreisförmigen Plättchen (2) besteht, das konzentrisch in eine Scheibe (3) eingebettet ist, wobei das Plättchen und die Scheibe aus verschiedenen Metallen oder aus verschiedenen Metalllegierungen ausgeführt sind.

3. Vorrichtung nach einem der Ansprüche 1 oder 2, **dadurch gekennzeichnet, dass** der mittlere Teil (2) aus Kupfer ausgeführt ist, während der Umfangsteil (3) aus Zink ausgeführt ist, oder umgekehrt.

4. Vorrichtung nach einem der Ansprüche 1 bis 3, **dadurch gekennzeichnet, dass** die Komponenten (2, 3) der flachen Platte (1) in einer gleichen Ebene angeordnet sind.

5. Vorrichtung nach einem der Ansprüche 1 bis 4, **dadurch gekennzeichnet, dass** sie in einer Sohle (4) untergebracht ist, die geeignet ist, herausnehmbar in einen Schuh eingelegt zu werden

6. Vorrichtung nach einem der Ansprüche 1 bis 4, **dadurch gekennzeichnet, dass** sie in einem Zwangsmittel (4) untergebracht ist, das aus einem üblichen, zum Anlegen an einen Teil des Körpers von lebenden Organismen bestimmten Gebrauchsartikel gebildet ist, wie beispielsweise Schuhe, Gürtel, Korsette, Bandagen, Verbände.

7. Vorrichtung nach einem der Ansprüche 1 bis 6, **dadurch gekennzeichnet, dass** ihre angelegte oder zum Anlegen an ein Zwangsmittel (4) bestimmte Seite mit einem Film aus haftendem Material (5) oder einem selbstklebendem Film (7) überzogen ist.

8. Vorrichtung nach einem der Ansprüche 1 bis 7, **dadurch gekennzeichnet, dass** ihre Seite, die dazu bestimmt ist, in Richtung auf den zu stimulierenden Hautbereich ausgerichtet zu werden, mit einem Film aus undurchlässigem Material (6) überzogen ist, der auf der Seite beispielsweise mittels eines Klebstoffs oder Neopren befestigt ist.

9. Aus einer Sohle (4) gebildete Vorrichtung, die dazu bestimmt ist, unter der Fußsohle angelegt zu werden, umfassend zwei übereinander angeordnete Schichten (4a, 4b), zwischen denen die Vorrichtung gemäß einem der Ansprüche 1 bis 8 aufgenommen ist.

10. Vorrichtung nach einem der Ansprüche 5 oder 9, **dadurch gekennzeichnet, dass** der Feldgenerator im mittleren Bereich der Sohle (4) positioniert ist.

## Claims

1. A device for biostimulating living organisms and/or neutralising the harmful effects of apparatuses, formed by a generator for generating electromagnetic fields which is in the form of a flat plate (1) comprising at least two metallic components (2, 3) constituted by different metals, **characterised in that** the flat plate (1) is constituted by a central portion (2) set in a peripheral portion (3), said central portion (2) and said peripheral portion (3) being made of metals.

2. A device according to claim 1 **characterised in that** the flat plate (1) is in the form of a small disc (1) constituted by a circular disc portion (2) set in concentric relationship in a ring (3), said disc portion and ring being made of different metals or different alloys of metals.

3. A device according to one of claims 1 and 2 **characterised in that** the central portion (2) is made of copper while the peripheral portion (3) is made of zinc or vice-versa.

4. A device according to any one of claims 1 to 3 **characterised in that** the components (2, 3) of the flat plate (1) are disposed in the same plane.

5. A device according to any one of claims 1 to 4 **characterised in that** it is accommodated in a sole (4) capable of being removably accommodated in a shoe.

6. A device according to any one of claims 1 to 4 **characterised in that** it is accommodated in a retaining means (4) formed by an article of common use which is intended to be applied against a part of the body of the living organisms such as shoes, belts, corsets, bandages or dressings.

7. A device according to any one of claims 1 to 6 **characterised in that** its face which is applied or intended to be applied to a retaining means (4) is covered with a film of adhesive material (5) or a self-adhesive film (7).

8. A device according to any one of claims 1 to 7 **characterised in that** its face which is intended to be oriented in the direction of the skin zone to be stimulated is covered by a film of impermeable material (6) fixed to said face for example by means of a glue or neoprene.

9. A device formed by a sole (4) intended to be applied under the sole of the feet comprising two superposed layers (4a, 4b), between which is incorporated the device according to any one of claims 1 to 8.

10. A device according to one of claims 5 and 9 **characterised in that** the field generator is positioned in the central zone of the sole (4).
